# FASCICULE DE BREVET EUROPEEN

(11) **EP 1 694 658 B1**
(45) Date de publication et mention de la délivrance du brevet: **25.02.2009**
(21) Numéro de dépôt: 04805651.9
(22) Date de dépôt: 07.12.2004
(51) Int. Cl.: C07D 281/10, C07C 271/28

(54) **PROCEDE DE FABRICATION DE PRODUIT DE TYPE THIAZOLO [3,4,5-DE] [4,1] BENZOTHIAZEPINE**
VERFAHREN ZUR HERSTELLUNG EINES PRODUKTS VOM THIAZOLO [3,4,5-DE][4,1]BENZOTHIAZEPIN-TYP
METHOD FOR THE PRODUCTION OF A THIAZOLO [3,4,5-DE] [4,1]BENZOTHIAZEPINE-TYPE PRODUCT

(30) Priorité: 12.12.2003 FR 0314574
(43) Date de publication de la demande: 30.08.2006
(73) Titulaire: Aventis Pharma S.A., 92160 Antony (FR)
(72) Inventeur: MUTTI, Stéphane, F-94170 LE PERREUX SUR MARNE (FR); MALPART, Joel, 77 Allée Arnoul Greban, 45160 OLIVET (FR); LAVIGNE, Michel, F-91380 CHILLY MAZARIN (FR); CHEVE, Michel, F-91450 SOISY SUR MARNE (FR)
(74) Mandataire: Morel-Pécheux, Muriel
(86) Numéro de dépôt international: PCT/FR2004/003138
(87) Numéro de publication internationale: WO 2005/066146

(56) Documents cités:
- WO-A-99/05147

## Description

La présente invention concerne les procédés de préparation de composés de type thiazolo[3,4,5-de][4,1]benzothiazépine et en particulier du méthanesulfonate du 6,6-dioxyde de 2-imino-9-trifluorométhyl-4,5-dihydro-2H,7H-thiazolo[3,4,5-de][4,1]benzo-thiazépine et du (R,S) 6-oxyde de 2-imino-9-trifluorométhyl-4,5-dihydro-2H,7H-thiazolo[3,4,5-de][4,1]benzothiazépine ainsi que ces énantiomères et leurs sels pharmaceutiquement acceptables. Ces produits sont décrits dans la demande de brevet WO 99/05147. Ces produits sont connus et sont particulièrement utiles pour le traitement et/ou la prévention des convulsions et des maladies liées à la transmission glutamatergique. Ils sont notamment utiles pour traiter et/ou prévenir toutes les ischémies (telles l'ischémie focale ou globale) consécutives à des accidents vasculaires cérébraux tels que le stroke thromboembolique et hémorragique, un arrêt cardiaque, une hypotension artérielle, une intervention chirurgicale cardiaque, vasculaire ou pulmonaire ou une hypoglycémie sévére, dans le traitement des effets dus à une anoxie, qu'elle soit périnatale ou consécutive à une noyade, une haute pression ou à des lésions cérébro-spinales, pour traiter ou prévenir l'évolution de maladies neurodégénératives, de la chorée d'HUNTINGTON, de la maladie d'ALZHEIMER et autres démences, de la sclérose latérale amyotrophique ou d'autre maladies du motoneurone, de l'atrophie olivo-pontocérébelleuse et de la maladie de PARKINSON, vis-à-vis des manifestations épileptogènes (épilepsie) et/ou convulsives, pour le traitement des traumatismes cérébraux ou spinaux, des traumatismes liés à la dégénérescence de l'oreille interne ou de la rétine, du tinnitus, de l'anxiété, de la dépression, de la schizophrénie, du syndrome de TOURETTE, des encéphalopathies hépatiques, des troubles du sommeil, des désordres du déficit attentionnel, des troubles des conditions hormonales (excès de la sécrétion de HG ou HL, sécrétion de corticostérone), en tant qu'analgésiques, antiinflammatoires, antianorexiques, antimigraineux, antiémétiques et pour traiter les empoisonnements par des neurotoxines ainsi que les troubles neurologiques associés aux maladies virales telles que les méningites et encéphalites virales, le SIDA, la rage, la rougeole et le tétanos. Ces composés sont aussi utiles pour la prévention, la tolérance et la dépendance des symptômes d'abstinence aux drogues, à l'alcool et de l'inhibition de l'accoutumance et de la dépendance aux opiacés, barbituriques, amphétamine et benzodiazépines. Ils peuvent également être utilisés dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

La présente invention concerne le développement d'un procédé de synthèse des produits du méthanesulfonate du 6,6-dioxyde de 2-imino-9-trifluorométhyl-4,5-dihydro-2H,7H-thiazolo[3,4,5-de][4,1]benzothiazépine et du (R,S) 6-oxyde de 2-imino-9-trifluorométhyl-4,5-dihydro-2H,7H-thiazolo[3,4,5-de][4,1]benzothiazépine ainsi que ces énantiomères le méthanesulfonate du (S) 6-oxyde de 2-imino-9-trifluorométhyl-4,5-dihydro-2H,7H-thiazolo[3,4,5-de][4,1]benzothiazépine et le méthanesulfonate du (R) 6-oxyde de 2-imino-9-trifluorométhyl-4,5-dihydro-2H,7H-thiazolo[3,4,5-de][4,1]benzothiazépine compatible avec une production à grande échelle.

La demande de brevet WO99/05147 décrit une méthode de synthèse générale qui est difficilement transposable à l'échelle industrielle pour une production en grande quantité. La synthèse est décrite dans les exemples 10, 11 et 13 de la demande de brevet WO99/05147 ainsi que de manière très générale dans la partie procédé de la description.

La présente invention a permis d'optimiser en terme de nombre d'étape et de rendement la synthèse permettant ainsi une production à grande échelle qui n'était pas envisageable à partir des données et conditions opératoires développées dans la demande de brevet WO99/05147.

En effet le nouveau procédé de synthèse a permis de réduire le nombre d'intermédiaires isolés ainsi que de supprimer les purifications par chromatographie sur silice.

La méthode de synthèse développées dans la demande de brevet WO99/05147 consiste à obtenir le méthanesulfonate du 6,6-dioxyde de 2-imino-9-trifluorométhyl-4,5-dihydro-2H,7H-thiazolo[3,4,5-de][4,1]benzothiazépine en 9 étapes à partir de la 4-trifluorométhyl-aniline et selon le schéma (I) réactionnel ci dessous 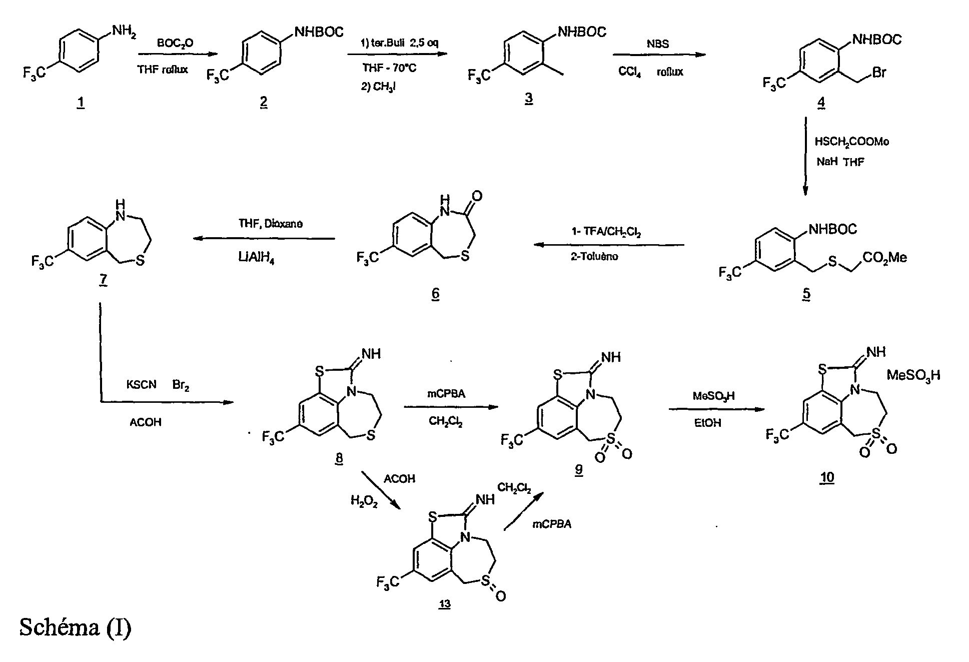 Le produit 1 est protégé selon des méthodes classiques avec un groupement BOC avec du di tert-butyl dicarbonate dans du THF à reflux pour obtenir le produit 2. Le produit 2 est transformé en produit 3 avec du tert butyl lithium qui est hautement instable. La fonction méthyle est bromé avec du N-bromosuccinimide pour obtenir le produit 4. La chaîne soufrée est introduite avec le thioglycoate de méthyle en présence d'hydrure de sodium pour obtenir l'intermédiaire 5. Cet intermédiaire 5 est cyclisé en présence d'acide trifluoroacétique . La fonction amide de l'intermédiaire 6 : 7-trifluorométhyl-1,5-dihydro-3H-[4,1]benzothiazépine-2-one est réduite en amine en présence de tétrahydroaluminate de lithium pour obtenir l'intermédiaire 7 : 7-trifluorométhyl-1,2,3,5-tétrahydro-[4,1]benzothiazépine. Le troisième cycle est introduit en présence de thiocyannate de potassium et de brome dans de l'acide acétique. L'intermédiaire clé 8: 2-imino-9-trifluorométhyl-4,5-dihydro-2H,7H-thiazolo[3,4,5-de][4,1]benzothiazépine est ainsi obtenu en 7 étapes.

L'intermédiaire 8 permet d'obtenir le produit final méthanesulfonate du 6,6-dioxyde de 2-imino-9-trifluorométhyl-4,5-dihydro-2H,7H-thiazolo[3,4,5-de][4,1]benzo-thiazépine mais aussi le (R,S) 6-oxyde de 2-imino-9-trifluorométhyl-4,5-dihydro-2H,7H-thiazolo[3,4,5-de][4,1]benzothiazépine ainsi que ces énantiomères.

La présente invention a permis d'optimiser chacune de ces étapes selon le schéma (II) réactionnel et en particulier d'en diminuer le nombre d'étape de synthèse et d'utiliser d'autres intermédiaires de synthèse, des réactifs industriels et de réaliser la cyclisation du 2° cycle directement en une étape et tout cela en supprimant les purifications par chromatographie.

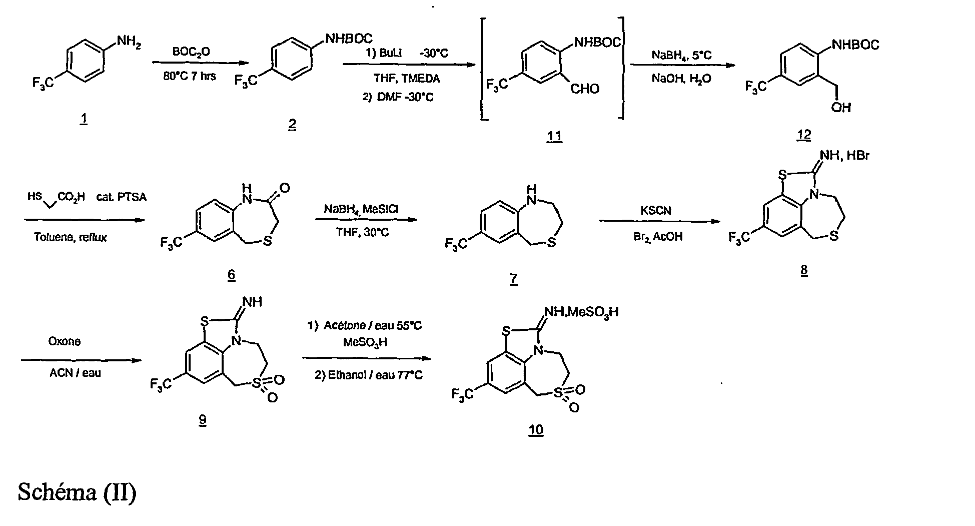

Le procédé de préparation du produit 10 du méthanesulfonate du 6,6-dioxyde de 2-imino-9-trifluorométhyl-4,5-dihydro-2H,7H-thiazolo[3,4,5-de][4,1]benzothiazépine caractérisé en ce que
a) la 4-trifluorométhylaniline 1 est protégé en présence de di-tert-butyl dicarbonate dans du monochlorobenzene à reflux puis,
b) le 4-trifluorométhyl phénylcarbamate de *tert*-butyle 2 isolé à l'étape précédente est transformé en 2-hydroxyméthyl-4-trifluorométhyl phénylcarbamate 12 sans isolement de l'intermédiaire 2-formyl-4-trifluorométhyl phénylcarbamate 11 puis,
c) le 2-hydroxyméthyl-4-trifluorométhyl phénylcarbamate 12 isolé à l'étape précédente est transformé directement en 7-trifluorométhyl-1,5-dihydro-3H-[4,1]benzothiazépine-2-one 6 en présence d'acide thioglycolique et d'une quantité catalytique d'acide p-toluène sulfonique puis,
d) le 7-trifluorométhyl-1,5-dihydro-3H-[4,1]benzothiazépine-2-one 6 isolé à l'étape précédente est réduit pour fournir du 7-trifluorométhyl-1,2,3,5-tétrahydro-[4,1]benzothiazépine 7 puis,
e) 7-trifluorométhyl-1,2,3,5-tétrahydro-[4,1]benzothiazépine isolé à l'étape précédente est transformé en 8 bromhydrate de 2-imino-9-trifluorométhyl-4,5-dihydro-2H,7H-thiazolo[3,4,5-de][4,1]benzothiazépine en présence de brome, de thiocyanate de potassium dans de l'acide acétique puis,
f) le bromhydrate de 2-imino-9-trifluorométhyl-4,5-dihydro-2H,7H-thiazolo[3,4,5-de][4,1]benzothiazépine est oxydé en 6,6-dioxyde de 2-imino-9-trifluorométhyl-4,5-dihydro-2H,7H-thiazolo[3,4,5-de][4,1]benzothiazépine 9 puis est transformé en présence d'acide méthane sulfonique en méthanesulfonate du 6,6-dioxyde de 2-imino-9-trifluorométhyl-4,5-dihydro-2H,7H-thiazolo[3,4,5-de] [4,1]benzo-thiazépine 10.

La première étape consiste en une protection de la fonction amine de 4-trifluorométhylaniline 1 avec du di tert-butyl dicarbonate dans du monochlorobenzene pendant 7 heures à reflux entre 78 et 82°C et préférentiellement 80°C. Le produit est cristallisé dans du méthylcyclohexane. Le produit 2 est obtenu avec un rendement de 88 %.

La seconde étape est jumelée avec la troisième car le produit intermédiaire 11 n'est pas isolé. Le produit 2 est mis en solution dans le tétrahydrofurane (THF) anhydre et la solution est refroidi entre -32 et -28° et préférentiellement -30°, la N, N, N', N' tetramethylethylediamine (TMEDA) est ajoutée ainsi que le n-Butyl lithium. Après 4 h 30 à -30°, du DMF (diméthylformamide) est ajouté. Le traitement est réalisé par coulée d'acide chlorhydrique 6N et le tout est chauffé à 20°C pendant 30 min. La phase aqueuse est éliminée et la phase organique est lavée avec de l'eau. Le produit 11 est ainsi conservé à froid dans du THF et est engagé directement dans l'étape suivante. A la solution du produit 11 dans du THF à 5°C, on ajoute un mélange de soude 0.1N et de NaBH₄ à 5°C. Après 3 heures à 5°C, une solution de HCl 6N est ajouté au milieu et le tout est réchauffé jusqu'à une température comprise entre 18 et 22°C et préférentiellement 20°C. Après décantation et lavage à l'eau, le solvant est évaporé et le produit est précipité en présence de méthylcyclohexane. Le produit 12 est obtenu avec un rendement de 69 % sur 2 étapes.

La quatrième étape à partir de l'intermédiaire 12 consiste à former le deuxième cycle soufré et obtenir le produit 6. L'intermédiaire 12 en solution dans du toluène avec de l'acide thioglycolique est ajouté à une solution de toluène et d'acide p-toluène sulfonique au reflux. Le tout est maintenu au reflux. L'eau est éliminée par un DeanStark au cours de la réaction. En fin de réaction, le mélange est refroidi jusqu'à une température comprise entre 9 et 11°C et préférentiellement 10°C et de l'eau est ajoutée ainsi que de l'ammoniaque. Le mélange réactionnel est filtré et le précipité est lavé à l'eau puis séché pour obtenir le produit 6 avec un rendement de 84 %.

La cinquième étape à partir de l'intermédiaire 6 consiste à réduire la fonction amide en amine. Le produit 6 est mis en solution dans du THF et du NaBH₄ est ajouté. Le tout est chauffé jusqu'à une température comprise entre 28°C et 32°C et préférentiellement 30°C et le chlorotriméthylsilane est ajouté. Après 2 h à 30°C, le milieu est refroidi à 15°C. Un mélange eau/THF est ajouté puis de la soude à 30 % jusqu'à un pH 12 en 30 minutes. Après décantation, la phase organique est lavée avec de l'eau et une solution de NaCl. Le produit 7 est précipité en présence de méthanol, est isolé et séché. Le produit 7 est obtenu avec un rendement de 92 %.

La sixième étape à partir de l'intermédiaire 7 consiste à former le troisième cycle et obtenir le produit 8. L'intermédiaire 7 est solubilisé dans l'acide acétique en présence de potassium thiocyanate. Le tout est chauffé jusqu'à une température comprise entre 29 et 31°C et préférentiellement 30°C et une solution de brome dans l'acide acétique est coulée en 2 heures. Après 2 heures à 30°C, le milieu est hydrolysé par addition d'eau, puis refroidit jusqu'à une température comprise entre 19 et 21 °C et préférentiellement 20°C. Le produit est filtré, lavé par de l'acide acétique puis essoré. Le gâteau est ensuite mis en suspension dans un mélange eau-méthanol, puis chauffé au reflux pendant 2 heures. Après un traitement au noir L3S, les filtrats sont concentrés sous vide de façon à éliminer le méthanol. Le milieu est ensuite refroidit entre 4 et 6°C et préférentiellement 5°C et est agité 30 minutes avant d'être filtré, lavé et séché en étuve. Le produit 8 est obtenu avec un rendement de 57.1 %

La septième étape consiste à oxyder le sulfure, intermédiaire clé 8, en sulfone. Le produit 8 est mis en suspension dans un mélange d'eau et d'acétonitrile (proportion 12/88). Puis on additionne l'oxone par petites fractions en 30mn en maintenant la température en dessous d'une température comprise entre 23 et 27°C et préférentiellement 25°C. L'oxydation du sulfure en intermédiaire sulfoxyde est complète 10 mn après la fin d'addition et après 3 heures d'agitation l'intermédiaire sulfoxyde est oxydé en sulfone attendue. L'excès d'oxone est détruit par ajout d'une solution aqueuse de sulfite de sodium à 5% puis le milieu acide est neutralisé à pH 9 par addition de soude N. La bouillie est concentrée à une température comprise entre 58 et 62°C et préférentiellement 60°C sous vide pour chasser l'acétonitrile, la suspension aqueuse obtenue est filtrée à une température comprise entre 43 et 47°C et préférentiellement 45°C, le gâteau est lavé à l'eau puis séché pour obtenir la sulfone 9 avec un rendement de 92 %.

La huitième étape consiste à salifier le produit 9 puis à recristalliser le sel obtenu. La sulfone 9 est mise en suspension dans un mélange d'eau et d'acétone, la suspension est chauffée à 55°C, on coule alors en 10mn l'acide méthane sulfonique, le milieu passe en solution, du noir 3S est ajouté et la suspension obtenue est filtrée sur clarcel. Le filtrat est dilué par ajout-d'acétone et le milieu cristallise, on laisse la température revenir à 25°C puis on refroidit à 10°C, la bouillie est filtrée, le gâteau est lavé à l'acétone et séché sous vide. Le produit 10 brut est obtenu sous forme d'un solvate à 0,2 mole/mole d'acétone avec un rendement de 75 %.

La suspension du sel brut 10 dans l'éthanol est chauffée à 77°C puis de l'eau est ajoutée rapidement, le milieu passe en solution puis recristallise. Après maintien 15mn à 75°C on laisse la température revenir à 25°C puis on refroidit à 10°C, le produit 10 est isolé et séché. On obtient le produit 10 avec un rendement de recristallisation de 75% soit un rendement global d'étape 8 de 56 %.

Le (R,S) 6-oxyde de 2-imino-9-trifluorométhyl-4,5-dihydro-2H,7H-thiazolo[3,4,5-de][4,1]benzothiazépine ainsi que ces énantiomères peuvent être obtenus selon les deux modes opératoires décrits dans le schéma (III) à partir de l'intermédiaire 8.

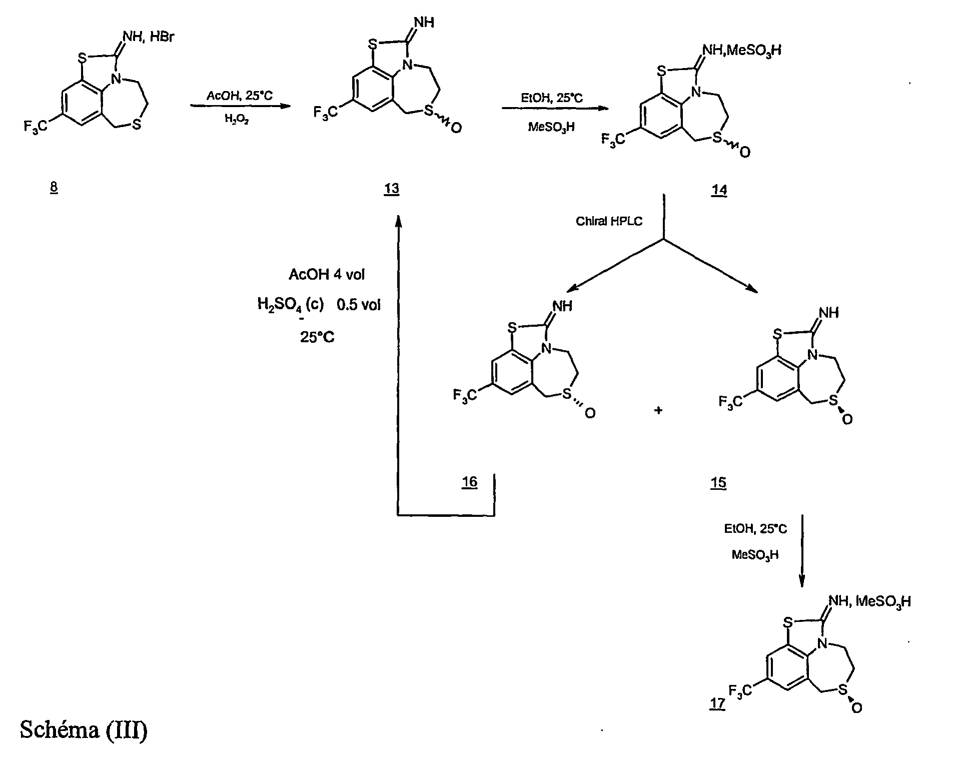

Le procédé de préparation de (S) 6-oxyde de 2-imino-9-trifluorométhyl-4,5-dihydro-2H,7H-thiazolo[3,4,5-de][4,1]benzothiazépine 16 **caractérisé en ce que**
i) le bromhydrate de 2-imino-9-trifluorométhyl-4,5-dihydro-2H,7H-thiazolo[3,4,5-de][4,1]benzothiazépine 8 est monooxydé sous la forme d'un racémique en (R,S) 6-oxyde de 2-imino-9-trifluorométhyl-4,5-dihydro-2H,7H-thiazolo[3,4,5-de][4,1]benzothiazépine 13 puis
ii) le (R,S) 6-oxyde de 2-imino-9-trifluorométhyl-4,5-dihydro-2H,7H-thiazolo[3,4,5-de][4,1]benzothiazépine est transformé en sel de méthanesulfonate de (R,S) 6-oxyde de 2-imino-9-trifluorométhyl-4,5-dihydro-2H,7H-thiazolo[3,4,5-de][4,1]benzothiazépine 14 puis
iii) le méthanesulfonate de (R,S) 6-oxyde de 2-imino-9-trifluorométhyl-4,5-dihydro-2H,7H-thiazolo[3,4,5-de][4,1]benzothiazépine est séparé par chromatographie chirale en (S) 6-oxyde de 2-imino-9-trifluorométhyl-4,5-dihydro-2H,7H-thiazolo[3,4,5-de][4,1]benzothiazépine 15 et (R) 6-oxyde de 2-imino-9-trifluorométhyl-4,5-dihydro-2H,7H-thiazolo[3,4,5-de][4,1]benzothiazépine 16 puis
iv) le (S) 6-oxyde de 2-imino-9-trifluorométhyl-4,5-dihydro-2H,7H-thiazolo[3,4,5-de][4,1]benzothiazépine est transformé en méthanesulfonate de (S) 6-oxyde de 2-imino-9-trifluorométhyl-4,5-dihydro-2H,7H-thiazolo[3,4,5-de][4,1]benzothiazépine 17
v) le (R) 6-oxyde de 2-imino-9-trifluorométhyl-4,5-dihydro-2H,7H-thiazolo[3,4,5-de][4,1]benzothiazépine 16 issu de l'étape iii) est recyclé en racémique (R,S) 6-oxyde de 2-imino-9-trifluorométhyl-4,5-dihydro-2H,7H-thiazolo[3,4,5-de][4,1]benzothiazépine qui est de nouveau séparé en (S) 6-oxyde de 2-imino-9-trifluorométhyl-4,5-dihydro-2H,7H-thiazolo[3,4,5-de][4,1]benzothiazépine et (R) 6-oxyde de 2-imino-9-trifluorométhyl-4,5-dihydro-2H,7H-thiazolo[3,4,5-de][4,1]benzothiazépine.

La synthèse du (R,S) 6-oxyde de 2-imino-9-trifluorométhyl-4,5-dihydro-2H,7H-thiazolo[3,4,5-de][4,1]benzothiazépine 13 est réalisée à partir du bromhydrate de 2-imino-9-trifluorométhyl-4,5-dihydro-2H,7H-thiazolo[3,4,5-de][4,1]benzothiazépine 8.

L'étape d'oxydation pour passer du produit 8 au produit 13 consiste à oxyder chimiquement la fonction sulfure en sulfoxide racémique. L'oxydation est réalisée par l'eau oxygénée dans l'acide acétique à 30°C. Un traitement réducteur au sulfite de sodium est suivi d'une coulée d'ammoniaque pour ramener le pH à 8,5. Le produit est ensuite filtré, lavé plusieurs fois par l'eau, l'éthanol puis séché en étuve à 40°C sous vide éjecteur. Le (R,S) 6-oxyde de 2-imino-9-trifluorométhyl-4,5-dihydro-2H,7H-thiazolo[3,4,5-de][4,1]benzothiazépine (13) racémique est obtenu avec un rendement de 94,4 %.

L'étape suivante consiste à transformer le (R,S) 6-oxyde de 2-imino-9-trifluorométhyl-4,5-dihydro-2H,7H-thiazolo[3,4,5-de][4,1]benzothiazépine en sel de methanesulfonate 14 (R,S) 6-oxyde de 2-imino-9-trifluorométhyl-4,5-dihydro-2H,7H-thiazolo[3,4,5-de][4,1]benzothiazépine. A une solution de (R,S) 6-oxyde de 2-imino-9-trifluorométhyl-4,5-dihydro-2H,7H-thiazolo[3,4,5-de][4,1]benzothiazépine 13 dans l'éthanol est additionné à 25°C l'acide methanesulfonique. Après un maintien d'une heure à cette température, le produit est filtré, lavé par de l'éthanol absolu puis séché en étuve sous vide à 40°C. Le méthanesulfonate du (R,S) 6-oxyde de 2-imino-9-trifluorométhyl-4,5-dihydro-2H,7H-thiazolo[3,4,5-de][4,1]benzothiazépine 14 est obtenu avec un rendement de 97,7 %.

L'étape suivante consiste à séparer les deux énantiomères du méthanesulfonate du (R,S) 6-oxyde de 2-imino-9-trifluorométhyl-4,5-dihydro-2H,7H-thiazolo[3,4,5-de][4,1]benzothiazépine 14 en 15 : (S) 6-oxyde de 2-imino-9-trifluorométhyl-4,5-dihydro-2H,7H-thiazolo[3,4,5-de][4,1]benzothiazépine et 16 (R): 6-oxyde de 2-imino-9-trifluorométhyl-4,5-dihydro-2H,7H-thiazolo[3,4,5-de][4,1]benzothiazépine par chromatographie sur phase stationnaire chirale de type Whelk^{®} OI,SS. L'éluant pour la chromatographie est préparé en mélangeant à 25°C dans des proportions bien définies l'eau, l'éthanol et la triéthylamine. L'éluant de balayage est préparé en mélangeant à 25°C dans des proportions bien définies l'eau, l'éthanol, le dimethylformamide et la triethylamine. Le produit méthanesulfonate du (R,S) 6-oxyde de 2-imino-9-trifluorométhyl-4,5-dihydro-2H,7H-thiazolo[3,4,5-de][4,1]benzo-thiazépine est solubilisé dans l'éluant et injecté sur la colonne. Les fractions contenant le produit sont concentrées sous vide. En fin de séparation l'élution est inversée pour récupérer l'autre énantiomère du 16 (R) 6-oxyde de 2-imino-9-trifluorométhyl-4,5-dihydro-2H,7H-thiazolo[3,4,5-de][4,1]benzothiazépine. Le produit 15 (S) 6-oxyde de 2-imino-9-trifluorométhyl-4,5-dihydro-2H,7H-thiazolo[3,4,5-de][4,1]benzothiazépine est obtenu avec un rendement de 45 %.

La séparation peut être aussi réalisée à partir de l'intermédiaire (R,S) 6-oxyde de 2-imino-9-trifluorométhyl-4,5-dihydro-2H,7H-thiazolo[3,4,5-de][4,1]benzothiazépine base libre sur phase Chiralpack^{®} AD avec un eluant contenant de l'acide trifluoroacétique. Dans ces conditions, le sel trifluoroacetate de (S) 6-oxyde de 2-imino-9-trifluorométhyl-4,5-dihydro-2H,7H-thiazolo[3,4,5-de][4,1]benzothiazépine est isolé. Après un retour à la base libre avec de l'ammoniaque, le sel de l'acide methanesulfonique est alors préparé.

L'étape suivante à partir du 15 (S) 6-oxyde de 2-imino-9-trifluorométhyl-4,5-dihydro-2H,7H-thiazolo[3,4,5-de][4,1]benzothiazépine consiste à préparer le sel de methanesulfonate. A une solution de (S) 6-oxyde de 2-imino-9-trifluorométhyl-4,5-dihydro-2H,7H-thiazolo[3,4,5-de][4,1]benzothiazépine dans l'éthanol est additionné à 25°C l'acide méthanesulfonique. Après un maintien d'une heure à cette température, le produit est filtré, lavé par de l'éthanol absolu puis séché en étuve sous vide à 40°C. Le méthanesulfonate du (S) 6-oxyde de 2-imino-9-trifluorométhyl-4,5-dihydro-2H,7H-thiazolo[3,4,5-de][4,1]benzothiazépine 17 est obtenu avec un rendement de 80%.

L'intermédiaire 16 (R) 6-oxyde de 2-imino-9-trifluorométhyl-4,5-dihydro-2H,7H-thiazolo[3,4,5-de][4,1]benzothiazépine est recyclé en 13 (R, S) 6-oxyde de 2-imino-9-trifluorométhyl-4,5-dihydro-2H,7H-thiazolo[3,4,5-de][4,1]benzothiazépine par racémisation en milieu acide. Le produit est solubilisé dans l'acide acétique en présence d'acide sulfurique à 25°C. Après traitement, le produit (R,S) 6-oxyde de 2-imino-9-trifluorométhyl-4,5-dihydro-2H,7H-thiazolo[3,4,5-de][4,1]benzothiazépine est isolé avec un rendement de 90 %.

La présente invention est illustrée dans les exemples de synthèse qui suivent :

### Synthèse du méthanesulfonate du 6,6-dioxyde de 2-imino-9-trifluorométhyl-4,5-dihydro-2H,7H-thiazolo[3,4,5-de][4,1]benzo-thiazépine

### Etape 1 :

Dans un réacteur de 5-litres sont chargés 1.6 kg de 4-trifluoromethylaniline 1 à 60.6 % dans le monochlorobenzene et 1.625 kgde di-ter-butyl dicarbonate. Le milieu réactionnel est chauffé à 80+/-2°C en 1H30 puis maintenu à cette température pendant 6 heures. 2.909 litres de méthylcyclohexane sont additionnés en 30 minutes puis le milieu est refroidit à 60°C en 30 minutes. A cette température 1 g d'amorce 2 est ajouté, puis le milieu est refroidit à 20+/-5°C en 2 heures. Après refroidissement à -5+/-1°C à la vitesse de 10°C/heure, le milieu est maintenu 1 heure puis filtré. Le gâteau est lavé par 970 ml de methylcyclohexane puis séché, étuve à 30-35°C sous vide(5 mbar) jusqu'à poids constant.

1.385 kg d'intermédiaire 2 sous forme de cristaux blancs sont ainsi obtenus avec un rendement de 88 %.

### Etape 2 :

Dans un réacteur de 1,5-litres sont chargés à température ambiante successivement, 400 ml de Tetrahydrofurane, et 50g d'intermédiaire 2. Le milieu réactionnel est refroidit à -30+/- 2°C. Quand la température atteint -20°C, 56,16 g de N,N,N,N-tetramethylethylenediamine sont chargés tout en poursuivant le refroidissement. Lorsque la température atteint -30°C, 161.81g de n-Buthyllithium en solution à 2,5 M/L dans le n-hexane sont coulés. Le milieu est agité à -30+/-2°C pendant 4 h 30 puis 31,09 g de Dimethylformamide sont alors additionnés en maintenant la température à -30+/-2°C. Après 30 minutes de réaction, 295 ml d'acide chlorhydrique 6N pré-refroidi sont coulés à -30°C. Le milieu est ensuite chauffer à 20°C en 1 heure puis agité 30 minutes à cette température avant d'être mis en décantation. Après avoir éliminé la phase aqueuse, la phase organique est lavée 2 fois par 125 ml d'eau déminéralisée. La phase organique est ensuite stockée à 0-5°C.

436,2g de solution tetrahydrofurane d'intermédiaire 11 à 11,47%p/p sont ainsi obtenus avec un rendement de 90,4 %.

### Etape 3 :

Dans un erlenmeyer de 100 ml, est chargé à température ambiante 24,1 ml de sodium hydroxyde 0,1N. Après refroidissement à 5+/-2°C, 4,43g de sodium borohydrure sont additionnés. Cette solution est coulée en 1h30 à 5+/-2°C dans un réacteur de 2 litres contenant 436,2 g de solution Tetrahydrofurane à 11,47%p/p de l'intermédiaire 11. Le milieu est ensuite maintenu à cette température pendant 3 heures. Le pH du milieu est ramené à PH=4 par coulée à 5°C de 21,5 ml d'acide chlorhydrique 6N. Après 15 minutes d'agitation, le milieu est chauffé à 20+/-2°C, puis les phases sont décantées. La phase organique décantée est ensuite lavée par 70 ml d'eau. La phase organique est ensuite concentrée sous 80 mbars sans dépasser 35°C. 200 ml de Methylcyclohexane sont alors ajoutés au concentrât est la distillation sous pression réduite est poursuivie. Le milieu est ensuite refroidi à -5+/-2°C puis filtré. Le gâteau est lavé par 2 fois 19 ml de methylcyclohexane, puis essorer et séché en étuve sous vide à 30°C.

38,5 g de produit 12 sont ainsi obtenus sous forme de cristaux blancs avec un rendement de 69 % (calculé sur 2 étapes).

### Etape 4 :

Dans un réacteur de 2-litres sont chargés successivement 400 ml de toluène et 53,31 g d'acide p-toluène sulfonique. Le milieu est chauffé au reflux et la solution constituée de 600 ml de toluène, 200g d'intermédiaire 12 et 96,82 g d'acide thioglycolique est coulée en 2 heures. L'eau générée dans la réaction est éliminée grâce à un Dean Starck. La masse réactionnelle est ensuite refroidie à 10+/-1°C et 300 ml d'eau sont additionnés. 73 ml d'ammoniaque à 20 % sont coulés de façon à amener le pH à 8. Après refroidissement à 5°C, le milieu est filtré, lavé par 320 ml d'eau puis 320 ml de toluène. Le produit est séché en étuve à 40°C sous vide. 143,03 g de produit 6 : 7-trifluorométhyl-1,5-dihydro-3H-[4,1]benzothiazépine-2-one sous forme d'une poudre jaune pale sont isolés avec un rendement de84,2 %.

### Etape 5 :

Dans un réacteur de 2-litres, sont chargés successivement à température ambiante, 997 ml de Tetrahydrofurane, 100g d'intermédiaire 6 et 32,68 g de sodium borohydrure. Le mélange est chauffé à 30+/-2°C et 93,88g de chlorotriméthylsilane sont coulés en 1h30. Après un maintien de 2 heures à cette température, le milieu est refroidi à 15°C. 500 ml d'un mélange eau-tétrahydrofurane (65/35 v/v) est additionné en environ 2 h 30 sans dépasser une température maximum de 20°C. Le pH est amené progressivement en 30 minutes à 12 par coulée à 20°C d'environ 60 ml de sodium hydroxyde à 30 %. Après un maintien 30 minutes, le milieu est décanté. La phase organique est lavée 2 fois par 37 ml d'une solution de chlorure de sodium et 180 ml d'eau. La phase organique est concentrée sous vide sans dépasser une température de 30°C. En fin de concentration, 220 ml de méthanol sont coulés et le milieu est concentré une nouvelle fois. 207 ml d'eau sont alors coulés en 15 minutes à 20°C et le milieu est ensuite refroidit à 0°C. Après un maintien de 30 minutes, le milieu est filtré et le gâteau est lavé par 72 ml d'eau puis séché en étuve à 40°C sous vide.

87,67 g de produit 7 : 7-trifluorométhyl-1,2,3,5-tétrahydro-[4,1]benzothiazépine sont obtenus avec un rendement de 91.9 %.

### Etape 6 :

Dans un réacteur de 2.5-litres, sont chargés successivement 600 ml d'acide acétique, 100 g d'intermédiaire 7-trifluorométhyl-1,2,3,5-tétrahydro-[4,1]benzothiazépine et 94.46 g de potassium thiocyanate. L'ensemble est chauffé à 30+/-1°C en 2heures. La solution constituée de 77.28 g de brome dans 184.8 ml d'acide acétique est coulée en 2 heures à 30+/-1°C. Après un maintien de 2 heures à 30°C, 100 ml d'eau déminéralisée sont additionnés et le milieu est refroidi à 20+/-1°C en 30 minutes. Après un maintien de 30 minutes à cette température, le milieu est filtré. Le gâteau est lavé 2 fois avec 2*100 ml d'acide acétique. Après essorage, 253,6 g de produit 8 brut est recristallisé dans 2,6 litres d' un mélange eau - méthanol (20/8 v/v). Après 2 heures de reflux, 10 g de noir L3S et 50 ml du mélange eau - méthanol (20/80 v/v) sont ajoutés et le reflux est prolongé 2 heures supplémentaires. Le milieu est filtré sur un filtre thermostaté et le gâteau est lavé 2 fois par 2*200 ml du mélange eau - méthanol. Les jus de filtrations sont ensuite rechargées dans un réacteur de 2.5 litres et concentrés sous 15 mbar à une température de 45°C. Lorsque la teneur en méthanol est inférieure à 5 % p/p, le milieu est refroidit à 5+/-1°C en 1 heure. Après un maintien à 5°C pendant 30 minutes, le milieu est filtré et le gâteau est lavé successivement à 5°C par 100 ml d'un mélange eau-méthanol (60/40 v/v), puis 2 fois par 2*100 ml d'acide acétique. Le produit est ensuite séché en étuve à 40°C sous vide.

88.8 g de produit 2-imino-9-trifluorométhyl-4,5-dihydro-2H,7H-thiazolo[3,4,5-de][4,1]benzothiazépine sous forme d'un solide blanc sont obtenus avec un rendement de 57.1 %.

### Etape 7

Dans un tricol de 6 litres agité, on charge 9,3g de 2-imino-9-trifluorométhyl-4,5-dihydro-2H,7H-thiazolo[3,4,5-de][4,1]benzothiazépine (sous forme de bromhydrate) 8, 164ml d'acétonitrile et 22ml d'eau (88/12). Sur la suspension obtenue on ajoute en 30mn par petites fractions, 24,6g d'oxone en maintenant la température du milieu < à 25°C ± 2°C (bain d'eau à 10°C) Au cours de l'addition la suspension épaissit et vire du blanc au jaune.

Une HPLC pratiquée 10mn après la fin d'addition montre que tout le 2-imino-9-trifluorométhyl-4,5-dihydro-2H,7H-thiazolo[3,4,5-de][4,1]benzothiazépine a été oxydé en intermédiaire sulfoxyde. Après 3 heures d'agitation l'intermédiaire sulfoxide est oxydé en sulfone attendue 6,6-dioxyde de 2-imino-9-trifluorométhyl-4,5-dihydro-2H,7H-thiazolo[3,4,5-de][4,1]benzo-thiazépine 9.

L'excès d'oxone est détruit par addition de 50ml de sulfite de sodium en solution aqueuse à 5 % (p/v) jusqu'à ce que le test peroxyde soit négatif. Dès que l'excès d'oxydant est détruit la couleur de la bouillie passe du jaune au blanc. La suspension acide est alors neutralisée par ajout de 150 ml de soude N jusqu'à un pH = 9.

La suspension est concentrée à 60°C ± 2°C sous 40 mmHg pour chasser l'acétonitrile. La suspension aqueuse obtenue est filtrée à 45°C ± 2°C sur verre fritté, le gâteau est lavé sur filtre par 3 fois 100ml d'eau distillée, essoré à fond et séché 72 heures à 35°C ± 2°C sous 50mmHg.

7,4g de 6,6-dioxyde de 2-imino-9-trifluorométhyl-4,5-dihydro-2H,7H-thiazolo[3,4,5-de][4,1]benzo-thiazépine 9 sont obtenus avec un rendement de 92 %

### Etape 8

Dans un tricol agité de 6 litres on charge 308,5 g de 6,6-dioxyde de 2-imino-9-trifluorométhyl-4,5-dihydro-2H,7H-thiazolo[3,4,5-de][4,1]benzo-thiazépine 9, 926 ml d'acétone et 926ml d'eau distillée, la suspension obtenue est chauffée à 55°C ± 2°C à cette température on coule 65,9 ml d'acide méthane sulfonique en 10mn, le milieu passe en solution, on ajoute alors 3 g de noir L3S et on filtre à chaud sur verre fritté. Sur le filtrat maintenu à chaud on ajoute rapidement 2778 ml d'acétone. Le milieu cristallise dès le début d'addition, on laisse la température du milieu revenir à 25°C ± 2°C puis on refroidit à 10°C ± 2°C à l'aide d'un bain de glace. Le milieu est filtré sur verre fritté, le gâteau essoré est lavé sur filtre par 3 fois 400 ml d'acétone, essoré à fond et séché 56 heures à 40°C sous 20 mmHg en atmosphère humide d'eau.

On obtient 302g de méthanesulfonate du 6,6-dioxyde de 2-imino-9-trifluorométhyl-4,5-dihydro-2H,7H-thiazolo[3,4,5-de][4,1]benzo-thiazépine brut solvaté par 0,2 mole/mole d'acétone avec un rendement de 75 %

Dans un tricol agité de 500 ml on charge 16 g de méthanesulfonate du 6,6-dioxyde de 2-imino-9-trifluorométhyl-4,5-dihydro-2H,7H-thiazolo[3,4,5-de][4,1]benzo-thiazépine (solvaté par 0,2 mole/mole d'acétone) et 160 ml d'éthanol. La suspension est chauffée à 77°C ± 2°C (voisinage de l'ébullition) et on ajoute en une fois 24 ml d'eau distillée. Le milieu passe en solution puis recristallise immédiatement, on laisse agiter 15 mn à 75°C ± 2°C puis on laisse revenir à l'ambiante sous agitation. On refroidit la bouillie à 10°C ± 2°C, on filtre sur verre fritté et le gâteau essoré à fond est séché 20 heures à 35°C ± 2°C sous 20 mmHg.

On obtient 12g de méthanesulfonate du 6,6-dioxyde de 2-imino-9-trifluorométhyl-4,5-dihydro-2H,7H-thiazolo[3,4,5-de][4,1]benzo-thiazépine 10 avec un rendement de recristallisation de 75 % soit un rendement global d'étape 8 de 56 %.

La synthèse du (R,S) 6-oxyde de 2-imino-9-trifluorométhyl-4,5-dihydro-2H,7H-thiazolo[3,4,5-de][4,1]benzothiazépine 13 et des ces énantiomères ont été réalisés selon les conditions décrites ci dessous.

Dans un réacteur de 8-litres, sont chargés successivement 2,8 litres d'acide acétique, 282,27 g d'intermédiaire 8. Le milieu est chauffé à 30+/-1°C et 126,7 g d'eau oxygénée sont additionnés en 1 heure. Après un maintien de 3 heures à cette température, la température est ramenée à 20+/-1°C. 14, 67 g de sulfite de sodium sont ajoutés lentement et après un maintien de 30 mn, 4,55 litres d'ammoniaque sont coulés. Après un maintien d'une heure, le milieu est filtré. Le gâteau est lavé successivement par 282,3 ml d'eau puis 5-,5 ml d'éthanol absolu. Le produit est séché en étuve à 40°C sous vide.

224,37g de produit 13 (R,S) 6-oxyde de 2-imino-9-trifluorométhyl-4,5-dihydro-2H,7H-thiazolo[3,4,5-de][4,1]benzothiazépine 13 sont obtenu sous forme d'une poudre blanche avec un rendement de 94,4 %.

Dans un réacteur de 2,5-litres, sont chargés à 25°C, 1012 ml d'éthanol absolu et 101,17 g d'intermédiaire 13 (R,S) 6-oxyde de 2-imino-9-trifluorométhyl-4,5-dihydro-2H,7H-thiazolo[3,4,5-de][4,1]benzothiazépine. 34,01 g d'acide méthanesulfonique sont alors coulés en 30 mn en maintenant la température à 25+/-1°C. Après une heure d'agitation, le pH est contrôlé et le milieu est filtré. Le gâteau est lavé par 50,6 ml d'éthanol absolu puis séché en étuve à 40°C sous vide. 129,64g de produit de 14 du méthanesulfonate de (R,S) 6-oxyde de 2-imino-9-trifluorométhyl-4,5-dihydro-2H,7H-thiazolo[3,4,5-de][4,1]benzothiazépine sous forme d'une poudre blanche sont isolés avec un rendement de 97,7 %.

90 g d'intermédiaire 14 méthanesulfonate de (R,S) 6-oxyde de 2-imino-9-trifluorométhyl-4,5-dihydro-2H,7H-thiazolo[3,4,5-de][4,1]benzothiazépine racémique sont mis en solution dans l'éluant constitué d'un mélange de 1,2 litres d'eau et 1,2 litres d'éthanol. La solution est filtrée avant d'être injectée sur la colonne par fraction. La colonne LC80 est packée à 25°C avec 1,2 kg de phase stationnaire Whelk^{®}-01 (S,S) dans 1,8 litres d'éthanol absolu. L'élution est réalisée avec un mélange constitué 18,7 litres d'eau, 28,1 litres d'éthanol absolu et 94 ml de triéthylamine. Alternativement, l'élution peut être réalisée avec un mélange constitué d'eau, d'éthanol absolu et de triéthylamine dans les proportions 20/80/0,1. Les fractions renfermant le produit attendu (S) 6-oxyde de 2-imino-9-trifluorométhyl-4,5-dihydro-2H,7H-thiazolo[3,4,5-de][4,1]benzothiazépine 15 sont rassemblées et concentrées sous vide. Le précipité est filtré et lavé par de l'eau avant d'être séché en étuve à 30°C sous vide. 30.1 g de produit (S) 6-oxyde de 2-imino-9-trifluorométhyl-4,5-dihydro-2H,7H-thiazolo[3,4,5-de][4,1]benzothiazépine 15 sous forme de cristaux blancs sont isolés avec un rendement d'environ 45 %.

L'autre énantiomère (R) 6-oxyde de 2-imino-9-trifluorométhyl-4,5-dihydro-2H,7H-thiazolo[3,4,5-de][4,1]benzothiazépine peut être récupéré en inversant le sans d'élution et en éluant avec un mélange constitué 0,47 litre d'eau, 7,9 litres d'éthanol, 0,94 litre de dimethylformamide et 18,7 ml de triéthylamine.

Dans un réacteur de 2,5-litres, sont chargés à 25°C, 2494 ml d'éthanol absolu et 83,13 g d'intermédiaire (S) 6-oxyde de 2-imino-9-trifluorométhyl-4,5-dihydro-2H,7H-thiazolo[3,4,5-de][4,1]benzothiazépine. 28,98 g d'acide méthanesulfonique sont alors coulés en 30 mn en maintenant la température à 25+/-1 °C. Après 30 mn d'agitation, la solution est filtrée sur millipore puis amorcée avec de l'intermédiaire (S) 6-oxyde de 2-imino-9-trifluorométhyl-4,5-dihydro-2H,7H-thiazolo[3,4,5-de][4,1]benzo-thiazépine pur. L'agitation est maintenue une heure à 25µC puis le milieu est refroidi lentement en 2 heures à 10°C. le milieu est ensuite concentré partiellement sous vide jusqu'à atteindre le minimum agitable. Après avoir refroidi à 5°C en 1 heure, le précipité est filtré. Le gâteau est lavé par 90 ml d'éthanol absolu puis séché en étuve à 40°C sous vide. Le produit est tamisé sur une grille de 0,630 mm.

87,43g de produit de méthanesulfonate de (S) 6-oxyde de 2-imino-9-trifluorométhyl-4,5-dihydro-2H,7H-thiazolo[3,4,5-de][4,1]benzothiazépine sous forme d'une poudre blanche sont isolés avec un rendement de 80 %.

L'autre énantiomère (R) 6-oxyde de 2-imino-9-trifluorométhyl-4,5-dihydro-2H,7H-thiazolo[3,4,5-de][4,1]benzothiazépine 16 est recyclé en (R,S) 6-oxyde de 2-imino-9-trifluorométhyl-4,5-dihydro-2H,7H-thiazolo[3,4,5-de][4,1]benzothiazépine en le solubilisant dans l'acide acétique en présence d'acide sulfurique à 25°C. Après traitement pour neutraliser l'acidité du milieu, le produit (R,S) 6-oxyde de 2-imino-9-trifluorométhyl-4,5-dihydro-2H,7H-thiazolo[3,4,5-de][4,1]benzothiazépine est isolé avec un rendement de 90 % qui de nouveau peut être séparé en (S) 6-oxyde de 2-imino-9-trifluorométhyl-4,5-dihydro-2H,7H-thiazolo[3,4,5-de][4,1]benzothiazépine et (R) 6-oxyde de 2-imino-9-trifluorométhyl-4,5-dihydro-2H,7H-thiazolo[3,4,5-de][4,1]benzothiazépine.

Une autre méthode pour séparer les deux énantiomères est exemplifiée ci dessous :

La séparation peut aussi être réalisée avec un système chromatographique différent à partir de l'intermédiaire (R,S) 6-oxyde de 2-imino-9-trifluorométhyl-4,5-dihydro-2H,7H-thiazolo[3,4,5-de][4,1]benzothiazépine 13 sur phase stationnaire Chiralpack^{®} AD avec un éluant comportant de l'acide trifluoroacetique. Les fractions renfermant le produit attendu rassemblées et concentrées sous vide. Le produit est isolé sous forme de son sel de trifuoroacétique

La séparation peut aussi être réalisée avec un système chromatographique différent à partir de l'intermédiaire 13 (R, S) 6-oxyde de 2-imino-9-trifluorométhyl-4,5-dihydro-2H,7H-thiazolo[3,4,5-de][4,1]benzothiazépine base libre sur phase stationnaire Chiralpack^{®} AD avec un éluant comportant de l'acide trifluoroacetique. Les fractions renfermant le produit attendu (S) 6-oxyde de 2-imino-9-trifluorométhyl-4,5-dihydro-2H,7H-thiazolo[3,4,5-de][4,1]benzothiazépine sont rassemblées et concentrées sous vide. Le produit (S) 6-oxyde de 2-imino-9-trifluorométhyl-4,5-dihydro-2H,7H-thiazolo[3,4,5-de][4,1]benzothiazépine est isolé sous forme de son sel de trifuoroacétique.

## Revendications

1. Procédé de préparation du méthanesulfonate du 6,6-dioxyde de 2-imino-9-trifluorométhyl-4,5-dihydro-2H,7H-thiazolo[3,4,5-de][4,1]benzothiazépine **caractérisé en ce que**
a) la 4-trifluorométhylaniline est protégé en présence de di-tert-butyl dicarbonate dans du monochlorobenzene à reflux puis,
b) le 4-trifluorométhyl phénylcarbamate de *tert*-butyle isolé à l'étape précédente est transformé en 2-hydroxyméthyl-4-trifluorométhyl phénylcarbamate sans isolement de l'intermédiaire 2-formyl-4-trifluorométhyl phénylcarbamate puis,
c) le 2-hydroxyméthyl-4-trifluorométhyl phénylcarbamate isolé à l'étape précédente est transformé directement en 7-trifluorométhyl-1,5-dihydro-3H-[4,1]benzothiazépine-2-one en présence d'acide thioglycolique et d'une quantité catalytique d'acide p-toluène sulfonique puis,
d) le 7-trifluorométhyl-1,5-dihydro-3H-[4,1]benzothiazépine-2-one isolé à l'étape précédente est réduit pour fournir du 7-trifluorométhyl-1,2,3,5-tétrahydro-[4,1]benzothiazépine puis,
e) 7-trifluorométhyl-1,2,3,5-tétrahydro-[4,1]benzothiazépine isolé à l'étape précédente est transformé en bromhydrate de 2-imino-9-trifluorométhyl-4,5-dihydro-2H,7H-thiazolo[3,4,5-de][4,1]benzothiazépine en présence de brome, de thiocyanate de potassium dans de l'acide acétique puis,
f) le bromhydrate de 2-imino-9-trifluorométhyl-4,5-dihydro-2H,7H-thiazolo[3,4,5-de][4,1]benzothiazépine est oxydé en 6,6-dioxyde de 2-imino-9-trifluorométhyl-4,5-dihydro-2H,7H-thiazolo[3,4,5-de][4,1]benzothiazépine puis est transformé en présence d'acide méthane sulfonique en méthanesulfonate du 6,6-dioxyde de 2-imino-9-trifluorométhyl-4,5-dihydro-2H,7H-thiazolo[3,4,5-de][4,1]benzo-thiazépine

2. Procédé selon la revendication 1 **caractérisé en ce que** le 4-trifluorométhyl phénylcarbamate de *tert*-butyle est obtenu par cristallisation dans du méthylcyclohexane.

3. Procédé selon la revendication 1 **caractérisé en ce que** le 2-hydroxyméthyl-4-trifluorométhyl phénylcarbamate est obtenu à partir du 2-formyl-4-trifluorométhyl phénylcarbamate, obtenu par réaction du n-butyl lithium sur le 4-trifluorométhyl phénylcarbamate de *tert*-butyle avec de du diméthylformamide, n'est pas isolé du milieu réactionnel.

4. Procédé de synthèse du 7-trifluorométhyl-1,5-dihydro-3H-[4,1]benzothiazépine-2-one selon la revendication 1 **caractérisé en ce qu'**il est obtenu à partir du 2-hydroxyméthyl-4-trifluorométhyl phénylcarbamate, d'acide thioglycolique et d'une quantité catalytique d'acide p-toluène sulfonique dans du toluène au reflux.

5. Procédé selon la revendication 1 **caractérisé en ce que** le 7-trifluorométhyl-1,5-dihydro-3H-[4,1]benzothiazépine-2-one est réduit en présence de NaBH₄ et de chlorotriméthylsilane.

6. Procédé selon la revendication 1 **caractérisé en ce que** le 6,6-dioxyde de 2-imino-9-trifluorométhyl-4,5-dihydro-2H,7H-thiazolo[3,4,5-de][4,1]benzothiazépine est obtenu à partir du bromhydrate de 2-imino-9-trifluorométhyl-4,5-dihydro-2H,7H-thiazolo[3,4,5-de][4,1]benzothiazépine en présence d'oxone dans l'acétonitrile et de l'eau.

7. Procédé de préparation de (S) 6-oxyde de 2-imino-9-trifluorométhyl-4,5-dihydro-2H,7H-thiazolo[3,4,5-de][4,1]benzothiazépine **caractérisé en ce que**
i) le bromhydrate de 2-imino-9-trifluorométhyl-4,5-dihydro-2H,7H-thiazolo[3,4,5-de][4,1]benzothiazépine est monooxydé sous la forme d'un racémique en (R,S) 6-oxyde de 2-imino-9-trifluorométhyl-4,5-dihydro-2H,7H-thiazolo[3,4,5-de][4,1]benzothiazépine puis
ii) le (R,S) 6-oxyde de 2-imino-9-trifluorométhyl-4,5-dihydro-2H,7H-thiazolo[3,4,5-de][4,1]benzothiazépine est transformé en sel de méthanesulfonate de (R,S) 6-oxyde de 2-imino-9-trifluorométhyl-4,5-dihydro-2H,7H-thiazolo[3,4,5-de][4,1]benzothiazépine puis
iii) le méthanesulfonate de (R,S) 6-oxyde de 2-imino-9-trifluorométhyl-4,5-dihydro-2H,7H-thiazolo[3,4,5-de][4,1]benzothiazépine est séparé par chromatographie chirale en (S) 6-oxyde de 2-imino-9-trifluorométhyl-4,5-dihydro-2H,7H-thiazolo[3,4,5-de][4,1]benzothiazépine et (R) 6-oxyde de 2-imino-9-trifluorométhyl-4,5-dihydro-2H,7H-thiazolo[3,4,5-de][4,1]benzothiazépine puis
iv) le (S) 6-oxyde de 2-imino-9-trifluorométhyl-4,5-dihydro-2H,7H-thiazolo[3,4,5-de][4,1]benzothiazépine est transformé en méthanesulfonate de (S) 6-oxyde de 2-imino-9-trifluorométhyl-4,5-dihydro-2H,7H-thiazolo[3,4,5-de][4,1]benzothiazépine
v) le (R) 6-oxyde de 2-imino-9-trifluorométhyl-4,5-dihydro-2H,7H-thiazolo[3,4,5-de][4,1]benzothiazépine issu de l'étape iii) est recyclé en racémique (R,S) 6-oxyde de 2-imino-9-trifluorométhyl-4,5-dihydro-2H,7H-thiazolo[3,4,5-de][4,1]benzothiazépine qui est de nouveau séparé en (S) 6-oxyde de 2-imino-9-trifluorométhyl-4,5-dihydro-2H,7H-thiazolo[3,4,5-de][4,1]benzothiazépine et (R) 6-oxyde de 2-imino-9-trifluorométhyl-4,5-dihydro-2H,7H-thiazolo[3,4,5-de][4,1]benzothiazépine.

8. Procédé selon la revendication 7 **caractérisé en ce que** le produit attendu (S) 6-oxyde de 2-imino-9-trifluorométhyl-4,5-dihydro-2H,7H-thiazolo[3,4,5-de][4,1]benzothiazépine 15 est élué lors de la chromatographie chirale par un mélange constitué d'eau, d'éthanol absolu et de triéthylamine dans les proportions 39/59/2.

9. Procédé selon la revendication 7 **caractérisé en ce que** le produit attendu (S) 6-oxyde de 2-imino-9-trifluorométhyl-4,5-dihydro-2H,7H-thiezolo[3,4,5-de][4,1]benzothiazépine 15 est élué lors de la chromatographie chirale à l'étape iii) par un mélange constitué d'eau, d'éthanol absolu et de triéthylamine dans les proportions 20/80/0,1.

10. Procédé selon la revendication 7 **caractérisé en ce que** le recyclage à l'étape v) est réalisé avec solubilisant le (R) 6-oxyde de 2-imino-9-trifluorométhyl-4,5-dihydro-2H,7H-thiazolo[3,4,5-de][4,1]benzothiazépine dans l'acide acétique en présence d'acide sulfurique.

11. Procédé selon la revendication 7 **caractérisé en ce que** oxydation de l'étape i) est réalisé avec de l'eau oxygénée dans de l'acide acétique.

12. Procédé de synthèse du bromhydrate de 2-imino-9-trifluorométhyl-4,5-dihydro-2H,7H-thiazolo[3,4,5-de][4,1]benzothiazépine **caractérisé en ce que**
a) la 4-trifluorométhylaniline est protégé en présence de di-tert-butyl dicarbonate dans du monochlorobenzene à reflux puis,
b) le 4-trifluorométhyl phénylcarbamate de *tert*-butyle isolé à l'étape précédente est transformé en 2-hydroxyméthyl-4-trifluorométhyl phénylcarbamate sans isolement de l'intermédiaire 2-formyl-4-trifluorométhyl phénylcarbamate puis,
c) le 2-hydroxyméthyl-4-trifluorométhyl phénylcarbamate isolé à l'étape précédente est transformé directement en 7-trifluorométhyl-1,5-dihydro-3H-[4,1]benzothiazépine-2-one en présence d'acide thioglycolique et d'une quantité catalytique d'acide p-toluène sulfonique puis,
d) le 7-trifluorométhyl-1,5-dihydro-3H-[4,1]benzothiazépine-2-one isolé à l'étape précédente est réduit pour fournir du 7-trifluorométhyl-1,2,3,5-tétrahydro-[4,1]benzothiazépine puis,
e) 7-trifluorométhyl-1,2,3,5-tétrahydro-[4,1]benzothiazépine isolé à l'étape précédente est transformé en bromhydrate de 2-imino-9-trifluorométhyl-4,5-dihydro-2H,7H-thiazolo[3,4,5-de][4,1]benzothiazépine en présence de brome, de thiocyanate de potassium dans de l'acide acétique.

13. A titre de produit intermédiaire, le 2-hydroxyméthyl-4-rifluorométhyl phénylcarbamate de *tert*-butyle

14. A titre de produit intermédiaire, le bromhydrate de 2-imino-9-trifluorométhyl-4,5-dihydro-2H,7H-thiazolo[3,4,5-de][4,1]benzothiazépine

15. A titre de produit intermédiaire, le sel trifluoroacetate de (S) 6-oxyde de 2-imino-9-trifluorométhyl-4,5-dihydro-2H,7H-thiazolo[3,4,5-de][4,1]benzothiazépine

## Claims

1. Process for preparing 2-imino-9-trifluoromethyl-4,5-dihydro-2H,7H-thiazolo[3,4,5-de][4,1]benzothiazepine 6,6-dioxide methanesulfonate, **characterized in that**
a) 4-trifluoromethylaniline is protected in the presence of di-tert-butyl dicarbonate in monochlorobenzene under reflux and then,
b) the *tert*-butyl 4-trifluoromethylphenylcarbamate isolated in the preceding step is converted to 2-hydroxymethyl-4-trifluoromethylphenylcarbamate without isolating the intermediate 2-formyl-4-trifluoromethylphenylcarbamate, and then
c) the 2-hydroxymethyl-4-trifluoromethylphenylcarbamate isolated in the preceding step is directly converted to 7-trifluoromethyl-1,5-dihydro-3H-[4,1]benzothiazepin-2-one in the presence of thioglycolic acid and a catalytic quantity of p-toluenesulfonic acid, and then
d) the 7-trifluoromethyl-1,5-dihydro-3H-[4,1]benzothiazepin-2-one isolated in the preceding step is reduced in order to give 7-trifluoromethyl-1,2,3,5-tetrahydro-[4,1]benzothiazepine, and then
e) 7-trifluoromethyl-1,2,3,5-tetrahydro-[4,1]-benzothiazepine isolated in the preceding step is converted to 2-imino-9-trifluoromethyl-4,5-dihydro-2H,7H-thiazolo[3,4,5-de][4,1]benzothiazepine hydrobromide in the presence of bromine, potassium thiocyanate in acetic acid, and then
f) the 2-imino-9-trifluoromethyl-4,5-dihydro-2H,7H-thiazolo[3,4,5-de][4,1]benzothiazepine hydrobromide is oxidized to 2-imino-9-trifluoromethyl-4,5-dihydro-2H,7H-thiazolo[3,4,5-de][4,1]benzothiazepine 6,6-dioxide and is then converted in the presence of methanesulfonic acid to 2-imino-9-trifluoromethyl-4,5-dihydro-2H,7H-thiazolo[3,4,5-de][4,1]benzothiazepine 6,6-dioxide methanesulfonate.

2. Process according to Claim 1, **characterized in that** *tert*-butyl 4-trifluoromethylphenylcarbamate is obtained by crystallization from methylcyclohexane.

3. Process according to Claim 1, **characterized in that** 2-hydroxymethyl-4-trifluoromethylphenylcarbamate is obtained from 2-formyl-4-trifluoromethylphenylcarbamate, obtained by the reaction of n-butyllithium on *tert*-butyl 4-trifluoromethylphenylcarbamate with dimethylformamide, is not isolated from the reaction medium.

4. Process for the synthesis of 7-trifluoromethyl-1,5-dihydro-3H-[4,1]benzothiazepin-2-one according to Claim 1, **characterized in that** it is obtained from 2-hydroxymethyl-4-trifluoromethylphenylcarbamate, thioglycolic acid and a catalytic quantity of p-toluenesulfonic acid in toluene under reflux.

5. Process according to Claim 1, **characterized in that** 7-trifluoromethyl-1,5-dihydro-3H-[4,1]benzothiazepin-2-one is reduced in the presence of NaBH₄ and chlorotrimethylsilane.

6. Process according to Claim 1, **characterized in that** 2-imino-9-trifluoromethyl-4,5-dihydro-2H,7H-thiazolo-[3,4,5-de][4,1]benzothiazepine 6,6-dioxide is obtained from 2-imino-9-trifluoromethyl-4,5-dihydro-2H,7H-thiazolo[3,4,5-de][4,1]benzothiazepine hydrobromide in the presence of oxone in acetonitrile and water.

7. Process for preparing (S)-2-imino-9-trifluoromethyl-4,5-dihydro-2H,7H-thiazolo[3,4,5-de][4,1]benzothiazepine 6-oxide, **characterized in that**
i) 2-imino-9-trifluoromethyl-4,5-dihydro-2H,7H-thiazolo[3,4,5-de][4,1]benzothiazepine hydrobromide is mono-oxidized in the form of a racemic to (R,S)-2-imino-9-trifluoromethyl-4,5-dihydro-2H,7H-thiazolo-[3,4,5-de][4,1]benzothiazepine 6-oxide, and then
ii) the (R,S)-2-imino-9-trifluoromethyl-4,5-dihydro-2H,7H-thiazolo[3,4,5-de][4,1]benzothiazepine 6-oxide is converted to a salt of (R,S)-2-imino-9-trifluoromethyl-4,5-dihydro-2H,7H-thiazolo[3,4,5-de][4,1]benzothiazepine 6-oxide methanesulfonate, and then
iii) the (R,S)-2-imino-9-trifluoromethyl-4,5-dihydro-2H,7H-thiazolo[3,4,5-de][4,1]benzothiazepine 6-oxide methanesulfonate is separated by chiral chromatography to (S)-2-imino-9-trifluoromethyl-4,5-dihydro-2H,7H-thiazolo[3,4,5-de][4,1]benzothiazepine 6-oxide and (R)-2-imino-9-trifluoromethyl-4,5-dihydro-2H,7H-thiazolo-[3,4,5-de][4,1]benzothiazepine 6-oxide, and then
iv) the (S)-2-imino-9-trifluoromethyl-4,5-dihydro-2H,7H-thiazolo[3,4,5-de][4,1]benzothiazepine 6-oxide is converted to (S)-2-imino-9-trifluoromethyl-4,5-dihydro-2H,7H-thiazolo[3,4,5-de][4,1]benzothiazepine 6-oxide methanesulfonate,
v) the (R)-2-imino-9-trifluoromethyl-4,5-dihydro-2H,7H-thiazolo[3,4,5-de][4,1]benzothiazepine 6-oxide derived from step iii) is recycled to a racemic (R,S)-2-imino-9-trifluoromethyl-4,5-dihydro-2H,7H-thiazolo[3,4,5-de][4,1]benzothiazepine 6-oxide which is again separated to (S)-2-imino-9-trifluoromethyl-4,5-dihydro-2H,7H-thiazolo[3,4,5-de][4,1]benzothiazepine 6-oxide and (R)-2-imino-9-trifluoromethyl-4,5-dihydro-2H,7H-thiazolo[3,4,5-de][4,1]benzothiazepine 6-oxide.

8. Process according to Claim 7, **characterized in that** the expected product (S)-2-imino-9-trifluoromethyl-4,5-dihydro-2H,7H-thiazolo[3,4,5-de][4,1] benzothiazepine 6-oxide 15 is eluted during the chiral chromatography with a mixture consisting of water, absolute ethanol and triethylamine in proportions of 39/59/2.

9. Process according to Claim 7, **characterized in that** the expected product (S)-2-imino-9-trifluoromethyl-4,5-dihydro-2H,7H-thiazolo[3,4,5-de][4,1] benzothiazepine 6-oxide 15 is eluted during the chiral chromatography in step iii) with a mixture consisting of water, absolute ethanol and triethylamine in proportions of 20/80/0.1.

10. Process according to Claim 7, **characterized in that** the recycling of step v) is carried out with solubilizing (R)-2-imino-9-trifluoromethyl-4,5-dihydro-2H,7H-thiazolo[3,4,5-de][4,1]benzothiazepine 6-oxide in acetic acid in the presence of sulfuric acid.

11. Process according to Claim 7, **characterized in that** oxidation of step i) is carried out with hydrogen peroxide in acetic acid.

12. Process for the synthesis of 2-imino-9-trifluoromethyl-4,5-dihydro-2H,7H-thiazolo[3,4,5-de]-[4,1]benzothiazepine hydrobromide, **characterized in that**
a) 4-trifluoromethylaniline is protected in the presence of di-tert-butyl dicarbonate in monochlorobenzene under reflux, and then
b) the *tert*-butyl 4-trifluoromethylphenylcarbamate isolated in the preceding step is converted to 2-hydroxymethyl-4-trifluoromethylphenylcarbamate without isolating the intermediate 2-formyl-4-trifluoromethylphenylcarbamate, and then
c) the 2-hydroxymethyl-4-trifluoromethylphenylcarbamate isolated in the preceding step is directly converted to 7-trifluoromethyl-1,5-dihydro-3H-[4,1]benzothiazepin-2-one in the presence of thioglycolic acid and a catalytic quantity of p-toluenesulfonic acid, and then
d) the 7-trifluoromethyl-1,5-dihydro-3H-[4,1]benzothiazepin-2-one isolated in the preceding step is reduced in order to give 7-trifluoromethyl-1,2,3,5-tetrahydro-[4,1]benzothiazepine, and then
e) the 7-trifluoromethyl-1,2,3,5-tetrahydro-[4,1]-benzothiazepine isolated in the preceding step is converted to 2-imino-9-trifluoromethyl-4,5-dihydro-2H,7H-thiazolo[3,4,5-de][4,1]benzothiazepine hydrobromide in the presence of bromine, potassium thiocyanate in acetic acid.

13. As intermediate product, *tert*-butyl 2-hydroxymethyl-4-trifluoromethylphenylcarbamate.

14. As intermediate product, 2-imino-9-trifluoromethyl-4,5-dihydro-2H,7H-thiazolo[3,4,5-de][4,1]benzothiazepine hydrobromide.

15. As intermediate product, the trifluoroacetate salt of (S)-2-imino-9-trifluoromethyl-4,5-dihydro-2H,7H-thiazolo[3,4,5-de][4,1]benzothiazepine 6-oxide.

## Patentansprüche

1. Verfahren zur Herstellung von 2-Imino-9-trifluormethyl-4,5-dihydro-2H,7H-thiazolo[3,4,5-de][4,1]-benzothiazepin-6,6-dioxid-methansulfonat, **dadurch gekennzeichnet, daß** man
a) 4-Trifluormethylanilin in Gegenwart von Di-tert-butyldicarbonat in Monochlorbenzol unter Rückfluß schützt,
b) das im vorhergehenden Schritt isolierte tert-Butyl-4-trifluormethylphenylcarbamat ohne Isolierung des Zwischenprodukts 2-Formyl-4-trifluormethylphenylcarbamat in 2-Hydroxymethyl-4-trifluormethylphenylcarbamat umwandelt,
c) das im vorhergehenden Schritt isolierte 2-Hydroxymethyl-4-trifluormethylphenylcarbamat in Gegenwart von Thioglykolsäure und einer katalytisch wirksamen Menge p-Toluolsulfonsäure direkt in 7-Trifluormethyl-1,5-dihydro-3H-[4,1]-benzothiazepin-2-on umwandelt,
d) das im vorhergehenden Schritt isolierte 7-Trifluormethyl-1,5-dihydro-3H-[4,1]benzothiazepin-2-on zu 7-Trifluormethyl-1,2,3,5-tetrahydro-[4,1]-benzothiazepin reduziert,
e) das im vorhergehenden Schritt isolierte 7-Trifluormethyl-1,2,3,5-tetrahydro-[4,1]benzothiazepin in Gegenwart von Brom und Kaliumthiocyanat in Essigsäure in 2-Imino-9-trifluormethyl-4,5-dihydro-2H,7H-thiazolo[3,4,5-de][4,1]-benzothiazepin-hydrobromid umwandelt und dann
f) das 2-Imino-9-trifluormethyl-4,5-dihydro-2H,7H-thiazolo[3,4,5-de][4,1]benzothiazepin-hydrobromid zu 2-Imino-9-trifluormethyl-4,5-dihydro-2H,7H-thiazolo[3,4,5-de][4,1]benzothiazepin-6,6-dioxid oxidiert und dann in Gegenwart von Methansulfonsäure in 2-Imino-9-trifluormethyl-4,5-dihydro-2H,7H-thiazolo[3,4,5-de][4,1]benzothiazepin-6,6-dioxid-methansulfonat umwandelt.

2. Verfahren nach Anspruch 1, **dadurch gekennzeichnet, daß** man das tert-Butyl-4-trifluormethylphenylcarbamat durch Kristallisation aus Methylcyclohexan erhält.

3. Verfahren nach Anspruch 1, **dadurch gekennzeichnet, daß** man das 2-Hydroxymethyl-4-trifluormethylphenylcarbamat aus 2-Formyl-4-trifluormethylphenylcarbamat, erhalten durch Umsetzung von n-Butyllithium mit tert-Butyl-4-trifluormethylphenylcarbamat mit Dimethylformamid, erhält, nicht aus dem Reaktionsmedium isoliert.

4. Verfahren zur Synthese von 7-Trifluormethyl-1,5-dihydro-3H-[4,1]benzothiazepin-2-on nach Anspruch 1, **dadurch gekennzeichnet, daß** man es aus 2-Hydroxymethyl-4-trifluormethylphenylcarbamat, Thioglykolsäure und einer katalytisch wirksamen Menge p-Toluolsulfonsäure in Toluol unter Rückfluß erhält.

5. Verfahren nach Anspruch 1, **dadurch gekennzeichnet, daß** man 7-Trifluormethyl-1,5-dihydro-3H-[4,1]-benzothiazepin-2-on in Gegenwart von NaBH₄ und Chlortrimethylsilan reduziert.

6. Verfahren nach Anspruch 1, **dadurch gekennzeichnet, daß** man das 2-Imino-9-trifluormethyl-4,5-dihydro-2H,7H-thiazolo[3,4,5-de][4,1]benzothiazepin-6,6-dioxid aus 2-Imino-9-trifluormethyl-4,5-dihydro-2H,7H-thiazolo[3,4,5-de][4,1]benzothiazepin-hydrobromid in Gegenwart von Oxone in Acetonitril und Wasser erhält.

7. Verfahren zur Herstellung von (S)-2-Imino-9-trifluormethyl-4,5-dihydro-2H,7H-thiazolo[3,4,5-de][4,1]benzothiazepin-6-oxid, **dadurch gekennzeichnet, daß** man
i) 2-Imino-9-trifluormethyl-4,5-dihydro-2H,7H-thiazolo[3,4,5-de][4,1]benzothiazepin-hydrobromid in Form eines Racemats zu (R,S)-2-Imino-9-trifluormethyl-4,5-dihydro-2H,7H-thiazolo[3,4,5-de][4,1]benzothiazepin-6-oxid monooxidiert,
ii) das (R,S)-2-Imino-9-trifluormethyl-4,5-dihydro-2H,7H-thiazolo[3,4,5-de][4,1]benzothiazepin-6-oxid in ein (R,S)-2-Imino-9-trifluormethyl-4,5-dihydro-2H,7H-thiazolo[3,4,5-de][4,1]benzothiazepin-6-oxid-Methansulfonatsalz umwandelt,
iii) das (R,S)-2-Imino-9-trifluormethyl-4,5-dihydro-2H,7H-thiazolo[3,4,5-de][4,1]benzothiazepin-6-oxid-methansulfonat durch chirale Chromatographie in (S)-2-Imino-9-trifluormethyl-4,5-dihydro-2H,7H-thiazolo[3,4,5-de][4,1]-benzothiazepin-6-oxid und (R)-2-Imino-9-trifluormethyl-4,5-dihydro-2H,7H-thiazolo[3,4,5-de][4,1]benzothiazepin-6-oxid trennt,
iv) das (S)-2-Imino-9-trifluormethyl-4,5-dihydro-2H,7H-thiazolo[3,4,5-de][4,1]benzothiazepin-6-oxid in (S)-2-Imino-9-trifluormethyl-4,5-dihydro-2H,7H-thiazolo[3,4,5-de][4,1]benzothiazepin-6-oxidmethansulfonat umwandelt und
v) das (R)-2-Imino-9-trifluormethyl-4,5-dihydro-2H,7H-thiazolo[3,4,5-de][4,1]benzothiazepin-6-oxid aus Schritt iii) zu racemischem (R,S)-2-Imino-9-trifluormethyl-4,5-dihydro-2H,7H-thiazolo[3,4,5-de][4,1]benzothiazepin-6-oxid rezykliert, welches man erneut in (S)-2-Imino-9-trifluormethyl-4,5-dihydro-2H,7H-thiazolo[3,4,5-de][4,1]benzothiazepin-6-oxid und (R)-2-Imino-9-trifluormethyl-4,5-dihydro-2H,7H-thiazolo[3,4,5-de][4,1]benzothiazepin-6-oxid trennt.

8. Verfahren nach Anspruch 7, **dadurch gekennzeichnet, daß** man das erwartete Produkt (S)-2-Imino-9-trifluormethyl-4,5-dihydro-2H,7H-thiazolo[3,4,5-de][4,1]benzothiazepin-6-oxid 15 bei der chiralen Chromatographie mit einer Mischung aus Wasser, absolutem Ethanol und Triethylamin in den Anteilen 39/59/2 eluiert.

9. Verfahren nach Anspruch 7, **dadurch gekennzeichnet, daß** man das erwartete Produkt (S)-2-Imino-9-trifluormethyl-4,5-dihydro-2H,7H-thiazolo[3,4,5-de][4,1]benzothiazepin-6-oxid 15 bei der chiralen Chromatographie in Schritt iii) mit einer Mischung aus Wasser, absolutem Ethanol und Triethylamin in den Anteilen 20/80/0,1 eluiert.

10. Verfahren nach Anspruch 7, **dadurch gekennzeichnet, daß** man die Rezyklierung in Schritt v) mit Solubilisierung des (R)-2-Imino-9-trifluormethyl-4,5-dihydro-2H,7H-thiazolo[3,4,5-de][4,1]benzothiazepin-6-oxids in Essigsäure in Gegenwart von Schwefelsäure durchführt.

11. Verfahren nach Anspruch 7, **dadurch gekennzeichnet, daß** man die Oxidation in Schritt i) mit Wasserstoffperoxid in Essigsäure durchführt.

12. Verfahren zur Synthese von 2-Imino-9-trifluormethyl-4,5-dihydro-2H,7H-thiazolo[3,4,5-de][4,1]-benzothiazepin-hydrobromid, **dadurch gekennzeichnet, daß** man
a) 4-Trifluormethylanilin in Gegenwart von Di-tert-butyldicarbonat in Monochlorbenzol unter Rückfluß schützt,
b) das im vorhergehenden Schritt isolierte tert-Butyl-4-trifluormethylphenylcarbamat ohne Isolierung des Zwischenprodukts 2-Formyl-4-trifluormethylphenylcarbamat in 2-Hydroxymethyl-4-trifluormethylphenylcarbamat umwandelt,
c) das im vorhergehenden Schritt isolierte 2-Hydroxymethyl-4-trifluormethylphenylcarbamat in Gegenwart von Thioglykolsäure und einer katalytisch wirksamen Menge p-Toluolsulfonsäure direkt in 7-Trifluormethyl-1,5-dihydro-3H-[4,1]-benzothiazepin-2-on umwandelt,
d) das im vorhergehenden Schritt isolierte 7-Trifluormethyl-1,5-dihydro-3H-[4,1]benzothiazepin-2-on zu 7-Trifluormethyl-1,2,3,5-tetrahydro-[4,1]-benzothiazepin reduziert,
e) das im vorhergehenden Schritt isolierte 7-Trifluormethyl-1,2,3,5-tetrahydro-[4,1]benzothiazepin in Gegenwart von Brom und Kaliumthiocyanat in Essigsäure in 2-Imino-9-trifluormethyl-4,5-dihydro-2H,7H-thiazolo[3,4,5-de][4,1]-benzothiazepin-hydrobromid umwandelt.

13. tert-Butyl-2-hydroxymethyl-4-trifluormethylphenylcarbamat als Zwischenprodukt.

14. 2-Imino-9-trifluormethyl-4,5-dihydro-2H,7H-thiazolo[3,4,5-de][4,1]benzothiazepin-hydrobromid als Zwischenprodukt.

15. (S)-2-Imino-9-trifluormethyl-4,5-dihydro-2H,7H-thiazolo[3,4,5-de][4,1]benzothiazepin-6-oxid-Trifluoracetatsalz als Zwischenprodukt.
